# EUROPÄISCHE PATENTSCHRIFT

(11) **EP 0 941 037 B1**
(45) Veröffentlichungstag und Bekanntmachung des Hinweises auf die Patenterteilung: **10.10.2001**
(21) Anmeldenummer: 97951969.1
(22) Anmeldetag: 25.11.1997
(51) Int. Cl.: A61B 17/70, A61B 17/72, A61B 17/74

(54) **PROPHYLAXE-IMPLANTAT GEGEN FRAKTUREN OSTEOPOROTISCH BEFALLENER KNOCHENSEGMENTE**
PROPHYLACTIC IMPLANT FOR PROTECTING BONE SEGMENTS AFFECTED BY OSTEOPOROSIS AGAINST FRACTURES
IMPLANT A USAGE PROPHYLACTIQUE SERVANT A METTRE DES SEGMENTS OSSEUX ATTEINTS D'OSTEOPOROSE A L'ABRI DE FRACTURES

(30) Priorität: 18.12.1996 DE 19652608
(43) Veröffentlichungstag der Anmeldung: 15.09.1999
(60) Teilanmeldung: 00101434.9
(73) Patentinhaber: ESKA Implants GmbH & Co., 23556 Lübeck (DE)
(72) Erfinder: GRUNDEI, Hans, D-23558 Lübeck (DE); THOMAS, Wolfram, I-00135 Roma (IT)
(74) Vertreter: Fuchs Mehler Weiss & Fritzsche
(86) Internationale Anmeldenummer: EP9706568
(87) Internationale Veröffentlichungsnummer: WO9826725

(56) Entgegenhaltungen:
- EP-A- 0 636 346
- WO-A-96/39974
- DE-A- 19 508 224
- DE-C- 4 208 247
- DE-U- 29 509 739
- US-A- 2 537 070

## Beschreibung

Die vorliegende Erfindung betrifft ein Prophylaxe-Implantat gegen Frakturen osteoporotisch befallener Knochensegmente.

Allein in Deutschland gibt es Millionen von Osteoporotikern, die also unter einer der häufigsten Erkrankungen des Bewegungsapparates leiden.

Bei der Osteoporose findet ein Verlust von Knochenmasse statt, der über den natürlichen altersbedingten Knochenabbau hinausgeht. Darüber hinaus verschlechtert sich die Qualität der Mikroarchitektur des Knochengewebes. Der Verlust an Knochenmasse, Knochenstruktur und Funktion führt dann zu klinischen Symptomen mit dauerhaften Beschwerden und Frakturen. Besonders weit verbreitet sind Schenkelhalsbrüche, Frakturen der Wirbelsäule und am Handgelenk.

Bei osteoporotischen Frakturen des Schenkelhalses kommt als Therapie heutzutage im wesentlichen nur die Totalresektion des Femurs und des natürlichen Acetabulums sowie die Implantation eines kompletten künstlichen Hüftgelenksystem in Betracht. Diese Systeme sind heute zwar ausgereift, der hierfür nötige Eingriff ist allerdings nach wie vor als ein schwerer Eingriff zu werten. Insbesondere kritisch ist aber darüber hinaus nach wie vor die Langzeitstabilität des Implantats im Patientenkörper. Einflüsse hierauf haben beispielsweise die Art des Implantates (zementfrei; zementiert), Alter und Konstitution des Patienten neben vielen anderen Parametern. Allein von daher wäre es wünschenswert, wenn die Totalresektion von Knochensegmenten so lang wie möglich auch bei Osteoporotikern hinausgezögert werden könnte oder gar völlig überflüssig werden würde.

Ansatzpunkte für eine Früherkennung des voraussichtlichen Traumas oder der Fraktur des betroffenen Knochensegmentes ergeben sich aus neuesten radiologischen Untersuchungen. So sind Zeichen der osteoporose-bedingten Veränderungen der Knochenstruktur radiologisch eindeutig feststellbar und interessanterweise ist der Zeitpunkt der voraussichtlichen Fraktur innerhalb eines Zeitraumes von bis zu einem Jahr mit erstaunlich hoher Genauigkeit vorhersagbar. Diese Erkenntnis bietet die Gelegenheit und die Chance, vor der Fraktur therapeutisch einzugreifen und die Osteoporose zum Stillstand zu bringen oder zurückzudrängen.

Ansatzweise ist versucht worden, das aufgezeigte Problem mit einer hohlen Hülse zu lösen, wie sie bekannt ist aus der US-A-2 537 070. Diese hohle Hülse wird in eine im Schenkelhals freigeräumte Bohrung gesetzt. Die Hülse verfügt über eine Anzahl von Durchbrechungen, durch welche hindurch das die Hülse nach der Implantation umgebende Knochenmaterial in das Innere der Hülse greifen kann und dort mit pulverisiertem Knochenmaterial in Kontakt treten kann. Schließlich soll das die Hülse umgebende Knochenmaterial sich mit dem pulverisierten Knochen in der Hülse verfestigen, da daß das Implantat langfristig als Armierungsimplantat wirken können soll.

Bei diesem bekannten Implantat wird es als nachteilig angesehen, daß die Kontaktfläche zwischen dem natürlichen Knochen und dem pulverisierten Knochenmaterial im Inneren der Hülse recht klein ist. Ist kein pulverisiertes Knochenmaterial im Inneren der Hülse vorgesehen, so lassen die Öffnungen bei dem bekannten Implantat ein nur sehr langsames Einsprossen von Knochenmaterial in das hohle Innere der Hülse zu. Hierdurch werden die Erfolge bei Einsatz des Implantats als Prophylaxe-Implantat geschmälert.

Vor diesem Hintergrund ist es die Aufgabe der vorliegenden Erfindung, ein verbessertes Prophylaxe-Implantat gegen Frakturen osteoporotisch befallener Knochensegmente anzugeben.

Gelöst wird die Aufgabe durch das im Anspruch 1 angegebene Prophylaxe-Implantat. Weitere vorteilhafte Ausführungsformen sind in den Unteransprüchen angegeben.

Erfindungsgemäß weist das Prophylaxe-Implantat einen dünnwandigen hohlen Armierungskörper mit einer solch großen Anzahl von Durchbrechungen in seiner Außenwandung auf, daß das Verhältnis der Gesamtflächen der Durchbrechungen zu der Gesamtoberfläche wenigstens 1:2 beträgt.

Der Armierungskörper kann im weitesten Sinne auch als Armierungshülse bezeichnet werden. Wesentlich ist, daß sein Inneres die Möglichkeit bietet, daß das Knochenmaterial durch die Durchbrechungen in sein Inneres wachsen kann bzw. in einem anderen Anwendungsfalle bioresorbierbares Material im Inneren des Armierungskörpers mit der Zeit resorbiert wird und ersetzt wird durch von außen hereingewachsenes Knochenmaterial. Der erfindungsgemäße Armierungskörper bildet somit einen Platzhalter für nachwachsendes gesundes Knochenmaterial anstelle des zuvor ausgeräumten osteoporotischen Knochenmaterials.

Operativ geht man nun so vor, daß die osteoporotisch befallenen Knochensegmente ausgefräst oder ausgebohrt werden und ein formlich an die Ausfräsung angepaßter Armierungskörper gemäß der vorliegenden Erfindung in das betreffende Knochensegment gesetzt wird. In geeigneter Weise wird dann das so präparierte Knochensegment wieder verschlossen. Dieser Anwendungsfalls ergibt einen also zunächst hohl bleibenden Armierungskörper im Inneren des betreffenden Knochensegmentes. Aufgrund der örtlichen Gegebenheiten und gegebenenfalls einer rauhen Oberfläche des Armierungskörpers wächst nun Knochenmaterial von außen in das Innere des Armierungskörpers hinein, und zwar so lange, bis dieser vollständig mit natürlicher Spongiosa gefüllt ist. Dieser Spongiosa haftet nun nicht mehr der Mangel osteoporotisch befallenen Knochenmaterials an; die Struktur und die Stabilität des befallenen Knochens ist somit wieder hergestellt.

Alternativ kann der Operateur so vorgehen, daß er das Innere des Armierungskörpers mit einem die Heilung fördernden Mittel-füllt, wobei hier insbesondere an Hydroxylapatit oder an eine alpha-Tricalciumphosphat-Keramik gedacht wird. Dieses Material wird gegebenenfalls unter Stopfen mittels eines geeigneten Werkzeuges im Inneren des Armierungskörpers komprimiert. Das Material ist bioresorbierbar, so daß nach einer Resorption des Materials im Laufe der Zeit natürliches Knochenmaterial in das Innere des Armierungskörpers hineinwächst.

Neben dem Haupteffekt, daß eine osteoporotisch bedingte Fraktur eines Knochensegmentes hinausgezögert oder völlig vermieden werden kann, ist anzuführen, daß auch die Belastung des Patienten während der Operation gegenüber jener bei der Implantation eines Totalersatzimplantates entscheidend reduziert wird, da das erfindungsgemäße Prophylaxe-Implantat im Rahmen eines ambulanten Eingriffs unter lediglich lokaler Anästhesie implantiert werden kann. Wenn also innerhalb des Vorhersagezeitraumes von bis zu einem Jahr von einer Fraktur des befallenen Knochensegmentes das erfindungsgemäße Phrophylaxe-Implantat appliziert wird, ist mit hoher Wahrscheinlichkeit von einer entscheidenden Entlastung des Patienten durch sonst sämtlich notwendig werdenden operativen Eingriffe auszugehen, da die Gelenke selbst nämlich in aller Regel zum Zeitpunkt der Vorsorgeuntersuchung noch intakt sind.

Gemäß einer ersten bevorzugten Ausführungsform besteht der Armierungskörper aus körperverträglichem Metall, und es ist abschnittsweise eine offenmaschige, dreidimensionale Raumnetzstruktur auf seiner Oberfläche vorgesehen. Die letztgenannte Raumnetzstruktur dient dazu, daß das umliegende Knochenmaterial durch die Struktur hindurchwachsen kann und so eine Hemmwirkung gegen ein Auswandern des Implantates aus der Ausfräsung in dem betreffenden Knochensegment ausübt. Im Extremfall, d.h. bei einem sehr großen Verhältnis der Gesamtflächen der Durchbrechungen zu der Gesamtoberfläche kann der Armierungskörper aus einer Art Metallnetz bestehen. Die dreidimensionale Raumnetzstruktur kann dann je nach Implantationsort an ausgesuchten Stellen der Oberfläche des Implantates vorgesehen sein.

Gemäß einer anderen vorteilhaften Ausführungsform ist vorgesehen, daß der Armierungskörper aus einem körperverträglichen formstabilen, aber gleichwohl noch elastischen Kunststoff besteht, wobei der Armierungskörper mittels eines in ihm integrierten Mechanismus unter Aufbringung einer Zugkraft stauchbar ist. Die bewußt durchgeführte Stauchung des Armierungskörpers hat den Sinn, ihn in situ zu halten, d.h. also an einer Auswanderung aus dem Implantationsort zu hindern. Die Stauchung ist möglich, da der Implantationskörper in diesem vorbeschriebenen Fall aus elastischem Kunststoff besteht.

Bei einer speziellen Ausbildung des erfindungsgemäßen Prophylaxe-Implantates als Schenkelhalsimplantat ist gemäß einer bevorzugten Ausführungsform vorgesehen, daß der Armierungskörper aus einer im wesentlichen zylindrischen Hülse besteht, welche distal mit einem balligen Kopfteil verschlossen ist und welche proximal mit einem Verschlußstück verschließbar ist. Auch eine in Richtung auf die Gelenkkugel des Hüftgelenks zulaufende konische Ausbildung der Hülse ist möglich.

Das proximal anzubringende Verschlußstück dient nach der gegebenenfalls vorgenommenen Befüllung des Hülseninneren mit beispielsweise Hydroxylapatit, einer alpha-Tricalciumphosphat-Keramik oder einer Mischung hieraus mit der zuvor ausgefrästen Spongiosa (s.o.) zum Verschließen der Hülse. Beispielsweise kann die Hülse mittels eines Schraubgewindes in eine Gewindebohrung in der Hülse geschraubt werden. Operativ kann es sinnvoll sein, vor dem Verschluß der Hülse eine Art Stopfen durch die Gewindebohrung in der Hülse zu führen, um das in dem Inneren befindliche Material zu komprimieren, so daß es an allen Stellen des künstlich erzeugten Knochenmaterials durch die Durchbrechungen in der Außenwandung der Hülse bis zu der Knochenwand vordringt, so daß der Resorptionsprozeß sofort beginnen kann.

Wenn die Hülse in diesem Fall aus Metall besteht, ist vorzugsweise das ballige Kopfteil wenigstens bereichsweise mit der Raumnetzstruktur versehen, um es - wie bereits ausgeführt - dem umliegenden Knochenmaterial zu ermöglichen, durch die Struktur hindurch zu wachsen zur dauerhaften Fixierung des Implantates in situ. Auch das proximale Verschlußstück kann wenigstens bereichsweise mit der Raumnetzstruktur versehen sein, und zwar aus demselben Grunde.

Bei der Ausführung des Implantates als Schenkelhalsimplantat aus körperverträglichem Kunststoff ist gemäß einer vorteilhaften Weiterbildung vorgesehen, daß im Inneren der Hülse zwischen dem Kopfteil und dem Verschlußstück mit diesen beiden Teilen verbunden eine Gewindespindel verläuft, mittels der die für die Stauchung notwendige Zugkraft auf Drehung der Gewindespindel hin erzeugbar ist. Operativ sieht es nun konkret so aus, daß der Operateur nach dem Einbringen der Hülse in den Knochenkanal mittels eines Werkzeuges, beispielsweise eines Schraubendrehers die Gewindespindel in Drehung versetzt, so daß diese das Bestreben hat, das Kopfstück in Richtung auf das Verschlußstück zu ziehen, wobei aufgrund der Elastizität des Kunststoffes die Hülse sich nach außen hin leicht ausbeult und so die Hemmungswirkung erzielt.

Alternativ hierzu kann vorgesehen sein, daß im Inneren der Hülse zwischen dem Kopfteil und dem Verschlußstück ein Zugseil gespannt ist, das vom Operateur her von extern weiter spannbar ist, beispielsweise durch Anwendung eines Schraubendrehers, um die zur Stauchung der Hülse aufzuwendende Zugkraft erzeugen zu können.

Das Zugseil kann hierbei im Kopfteil durch eine Zugplatte arretiert sein, wohingegen das Gegenstück am anderen Ende mit einer Gewindebohrung im Verschlußstück zusammenwirkt.

Gemäß einer noch weiteren vorteilhaften Weiterbildung kann vorgesehen sein, bei allen vorgeschriebenen Ausführungsformen die Außenwandung mit einem vom menschlichen Körper resorbierbaren Mittel zu beschichten. Dies erhöht die Akzeptanz des Implantates im Knochen und beschleunigt die Resorption, d.h. letztendlich die Einlagerung und Einorganisation von Knochenmaterial. Als Mittel kommen hierbei vorzugsweise wieder Hydroxylapatit oder eine alpha-Tricalciumsphosphat-Keramik zur Anwendung.

Gemäß einer Weiterentwicklung des Implantates ist vorgesehen, daß im Armierungskörper auf einem darin zentriert angeordneten magnetischen Kern eine elektrische Spule angeordnet ist, die von extern her anregbar ist.

Die vorliegende Erfindung macht sich die sogenannte magnetisch induzierte Elektrostimulation in der Orthopädie zunutze, wie sie ausführlich in der einschlägigen Fachliteratur beschrieben ist. Beispielsweise sei hingewiesen auf den Aufsatz "Magnetfeldtherapie und magnetisch induzierte Elektrostimulation in der Orthopädie", W. Kraus, DE-Z-Orthopäde (1984) 13:78 bis 92.

Die auf dem magnetischen Kern angeordnete Spule ist in dieser Konfiguration anzusehen als die Sekundärwicklung eines Transformators, dessen Primärwicklung von extern her an den Patienten gelegt wird. Ein niederfrequenter Wechselstrom in der Primärwicklung erzeugt eine ebenso niederfrequente Spannung an den Klemmen der Sekundärwicklung auf dem magnetischen Kern. In der Sekundärwicklung fließt dann über das Füllmaterial im Inneren des Armierungskörpers ein elektrischer Strom, welcher das Knochenwachstum in bekannter Weise stimuliert.

Als Resultat wird der Armierungskörper schneller von natürlicher Spongiosa durchwachsen, wodurch die Struktur und die Stabilität des befallenen Knochens deutlich schneller wiederhergestellt wird.

Gemäß einer vorteilhaften Weiterbildung kann vorgesehen sein, daß der magnetische Kern als Gewindespindel ausgebildet ist, welche dazu dient, die in diesem Falle aus Kunststoff bestehende zylindrische Hülse des Armierungskörpers durch Drehung der Gewindespindel zu stauchen, um so eine Wanderungsneigung des Implantates im Knochenkanal zu unterbinden.

Gemäß einer noch weiteren vorteilhaften Ausführungsform ist vorgesehen, daß das Kopfteil einen in das Innere des Armierungskörpers ragenden Fortsatz mit einer die zentrierte Lage des magnetischen Kerns sichernden Aufnahme aufweist. Das Kopfteil und der Fortsatz kann beispielsweise und bevorzugterweise aus einer geeigneten Keramik bestehen. Ist beispielsweise die Spitze des magnetischen Kerns kegelförmig ausgebildet, so ist die Aufnahme vorzugsweise komplementär hierzu in der Keramik vorgesehen.

Die Erfindung wird anhand einiger Ausführungsbeispiele anhand der Zeichnungsfiguren näher erläutert. Hierbei zeigt:
- Figur 1: eine schematische Ansicht der Implantationslage eines Implantates gemäß einer ersten Ausführungsform im Femur,
- Figur 2: den Hülsenteil des Implantates gemäß Figur 1 ohne Verschlußstück,
- Figur 3: eine weitere Ausführungsform des Implantates aus Kunststoff,
- Figur 4: eine noch weitere Ausführungsform des Implantates aus Kunststoff,
- Figur 5: die Ansicht des Implantates gemäß der Weiterentwicklung, teilweise weggebrochen,
- Figur 6: eine schematische Ansicht der Implantationslage des Implantates aus Figur 5 im Femurschenkelhals, und
- Figur 7: eine schematische Ansicht der Implantationslage des Implantates in einem Wirbelkörper.

In den Figuren sind gleiche Teile jeweils mit denselben Bezugszeichen versehen.

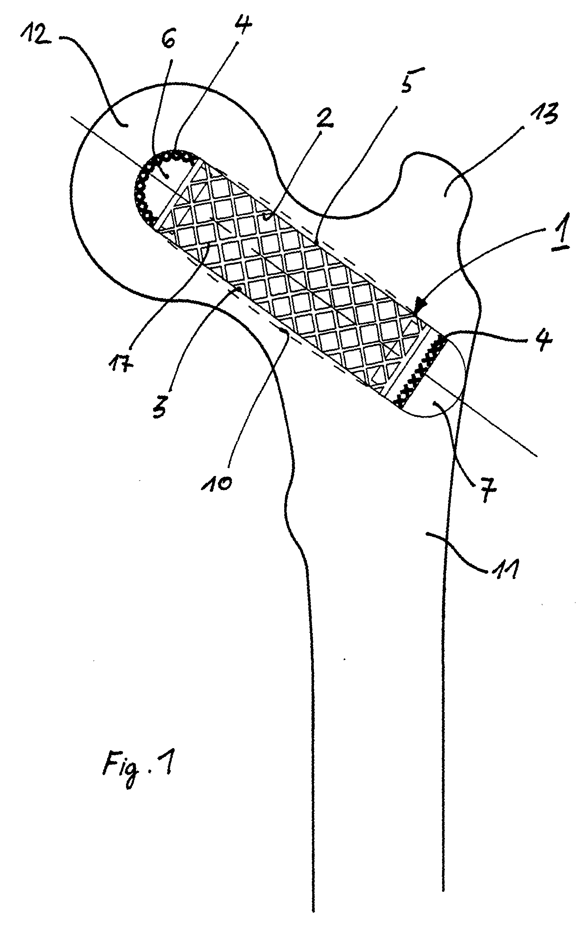

Figur 1 veranschaulicht die Einbaulage des Armierungskörpers 1 im Femur 11 eines Patienten, genauer gesagt im Schenkelhals. Der Armierungskörper 1 ist vorliegend als Schenkelhalsimplantat dargestellt, welches im wesentlichen aus der zylindrischen Hülse 5 besteht. Die Hülse 5 ist in einem in den Schenkelhals unterhalb des Trochanter major 13 eingebrachten Knochenkanal eingesetzt und bildet dort quasi einen Platzhalter für nachwachsendes gesundes Knochenmaterial. Hierzu weist die Hülse 5 Durchbrechungen 2 in der Außenwandung 3 auf. Bei dem dargestellten Implantat ist das Verhältnis der Gesamtflächen der Durchbrechungen 2 zu der Gesamtoberfläche des Implantates wesentlich größer als 1:2. Es handelt sich hierbei um ein Implantat, das gebildet ist aus einem metallischen Netz mit den Netzstegen 17.

Distal, d.h. in Richtung auf die Gelenkkugel 12 gesehen, ist die zylindrische Hülse 5 mit einem balligen Kopfteil 6 verschlossen. Das ballige Kopfteil 6 zeigt auf seiner Oberfläche bereichsweise eine offenmaschige dreidimensionale Raumnetzstruktur 4, durch welche nach einiger Zeit Knochenmaterial hindurchwächst, um so die Hülse 5 an einem Verrutschen oder Auswandern aus der Implantationslage zu hindern.

Proximal, also im Bereich der Anbringung der Bohrung oder des Knochenkanals im Femur 11 ist die Hülse 5 vorliegend verschlossen mit einem Verschlußstück 7, welches - wie das Kopfteil 6 - bereichsweise eine offenmaschige dreidimensionale Raumnetzstruktur aufweist, welche wiederum von Knochenmaterial durchwachsen wird zur Sicherung der Implantationslage.

Vorliegend ist die Außenwandung 3 der Hülse 5 mit einem bioresorbierbaren Mittel 10 beschichtet, welches beispielsweise Hydroxylapatit ist oder aus einer alpha-Tricalciumphosphat-Keramik besteht.

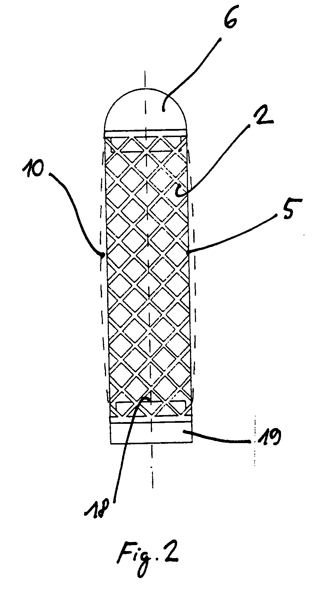

Figur 2 zeigt das Hülsenteil 5 des Implantates aus Figur 1, allerdings nicht im implantierten Zustand. Hier wird gesondert Bezug genommen auf die Durchbohrung 18 im Boden 19 der Hülse 5. Diese Durchbohrung 18 ist vorteilhaft eine Gewindebohrung, mit welcher das Verschlußstück 7 (Figur 1) verschraubt werden kann. Bevor dies jedoch unternommen wird, kann der Operateur nach seiner Wahl noch eine Art Stößel durch die Durchbohrung 18 führen, sofern vorher ein Füllmaterial in das Innere der Hülse eingefüllt worden ist. Dieses Füllmaterial kann aus Hydroxylapatit, einer alpha-Tricalciumphosphat-Keramik oder aus der Mischung von beiden oder allein mit Teilen der durch Ausfräsung entfernten Spongiosa bestehen. Der durch die Durchbohrung 18 zu rührende Stößel komprimiert diese Masse im Inneren der Hülse 5, so daß diese durch die Durchbrechungen 2 bis an die Wand des Knochenkanals gedrückt wird. Hierin kann eine heilungsfördernde Maßnahme gesehen werden, da nämlich das natürliche Knochenmaterial sofort in Kontakt mit dem bioresorbierbaren Stoff steht.

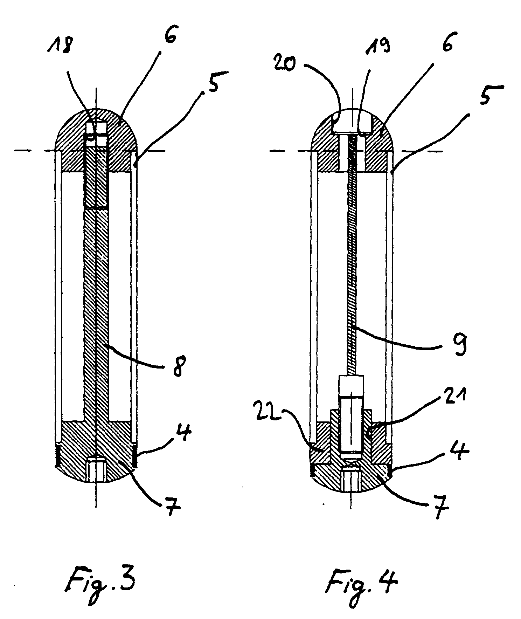

Figur 3 zeigt einen Schnitt durch ein Schenkelhalsimplantat in Form einer aus Kunststoff bestehenden zylindrischen Hülse 5, die distal wiederum mit dem balligen Kopfteil 6 verschlossen ist und proximal mit einem Verschlußstück 7. Um die Wanderungsneigung des Implantates im Knochenkanal zu unterbinden, ist vorliegend das Verschlußstück 7 mit einer Gewindespindel 8 versehen, welche in eine Gewindebohrung 18 im Kopfteil 6 greift. Auf Drehung des Verschlußstückes 7 kann der Operateur nun die Zugkräfte einstellen, mit welchen das Kopfstück 6 hin zum Verschlußstück 7 gezogen wird, wobei das elastische Kunststoffmaterial der Hülse 5 nachgibt und das Implantat so eine leichte bauchige Außenkontur annimmt. Hierdurch wird das umliegende Knochenfeld verdichtet, wodurch die Heilungschancen unter Wahrnehmung der Hemmsicherungswirkung erhöht werden.

Vorliegend ist nur noch am Verschlußstück 7 ein kleiner Bereich mit einer offenmaschigen dreidimensionalen Raumnetzstruktur belegt, um auch Mikrobewegungen dieses Teils nach Durchwachsen der Struktur mit Knochenmaterial zu verhindern.

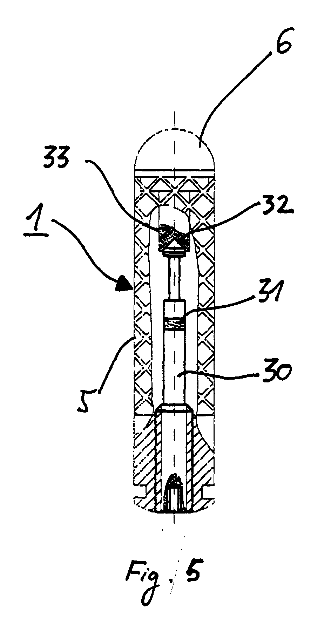

Figur 4 zeigt ebenfalls einen Schnitt durch eine weitere Ausführungsform. Vorliegend ist zwischen dem Kopfteil 6 und dem Verschlußstück 7 ein Zugseil 9 gespannt, wozu im Kopfteil 6 eine Zugplatte 19 in einer Bohrung 20 liegt, an welcher das Zugseil 9 befestigt ist. Der Verschlußstück 7 greift mit einem Gewindeschaft 21 durch eine Gewindehülse 22, die im Inneren und am Boden der Hülse 5 eingelassen ist und eine passende Gewindebohrung aufweist. Auf Drehung des Verschlußstückes 7 nun wird das Zugseil 9, welches in dem Gewindeschaft 21 verankert ist, weitergespannt, weswegen es - wie im Zusammenhang mit dem Ausführungsbeispiel gemäß Figur 3 erläutert - zu einer Ausbeulung der Hülse kommt, wodurch das Implantat im Knochenkanal fixiert wird. Auch vorliegend trägt das Schlußstück 7 in kleinen Abschnitten eine dreidimensionale offenmaschige Raumnetzstruktur, welche nach Durchwachsen mit Knochenmaterial für die Fixierung des Verschlußstückes 7 im Knochen sorgt.

Wie in Figur 5 schematisch dargestellt, ist der Armierungskörper 1 eine im wesentlichen hohle zylindrische Hülse 5. Darüber hinaus ist im Inneren der Hülse 5 ein magnetischer Kern zentriert angeordnet. Der magnetische Kern 30 trägt eine Spule 31, welche die Sekundärwicklung eines Transformators ist, der gebildet ist aus der Spule 31 und einer extern an den Patientenkörper heranzuführenden Primärwicklung oder Spule (nicht dargestellt), durch welche ein niederfrequenter Wechselstrom von ca. 0,2 bis 20 Hz geleitet wird, um die Therapie einzuleiten. Dieser Wechselstrom im Primärkreis erzeugt einen Strom im Sekundärkreis, nämlich in der Spule 31 und in dem zwischen beiden Spulenklemmen befindlichen Füllmaterial im Inneren der Hülse 5.

Der Kopfteil 6 der Hülse 5 weist einen in das Innere des Armierungskörpers 1 ragenden Fortsatz 33 auf, der an seinem stirnseitigen Ende eine komplementär zu der Spitze des magnetischen Kerns 30 ausgebildete Aufnahme 32 aufweist. Das Kopfteil 6 besteht vorzugsweise aus einer körperverträglichen Keramik.

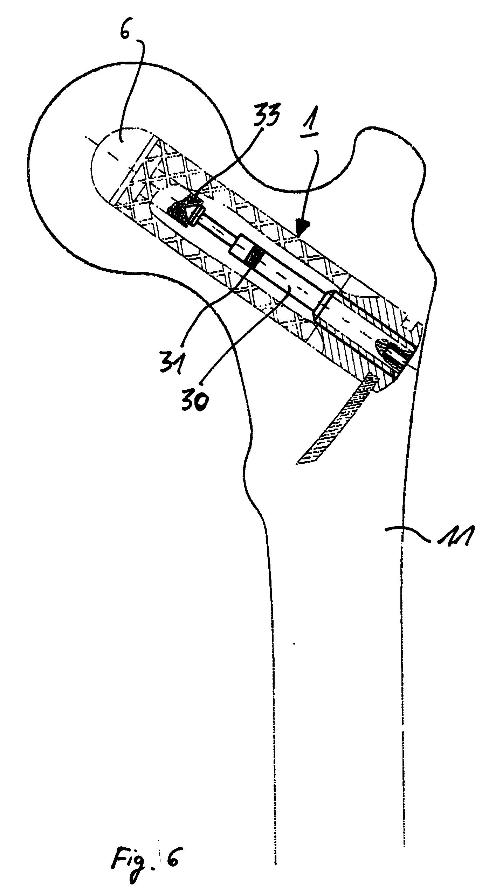

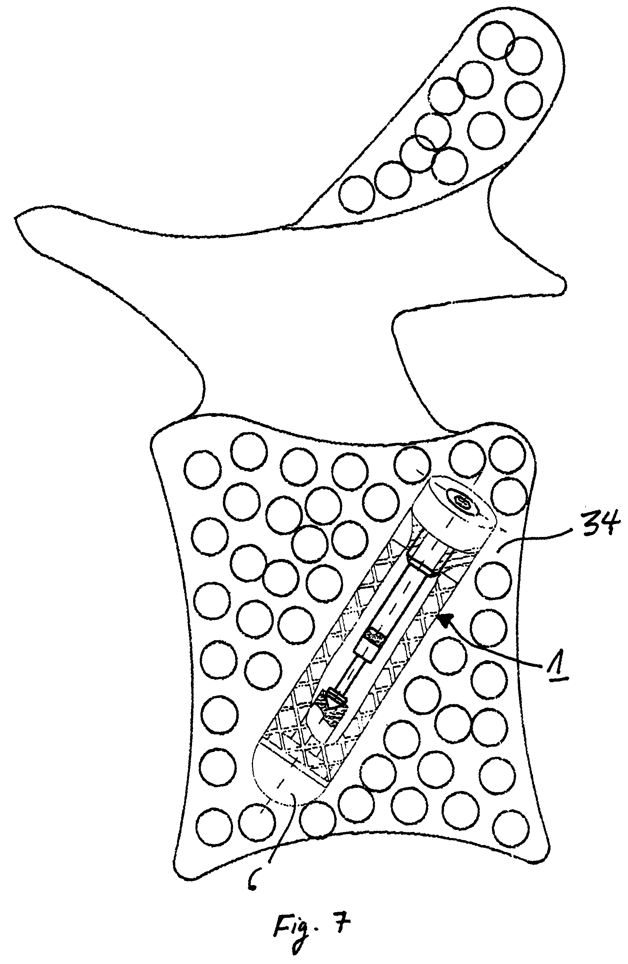

Figur 6 veranschaulicht zur Vervollständigung des Bildes die Implantationslage des Implantates im Schenkelhals des Femurs 11. Eine ähnliche Ausführungsform der Armierungshülse kann vorgesehen sein zum Einsatz in einem Wirbelkörper 34, wie dies in Figur 7 dargestellt ist. Freilich sind die Dimensionen andere als bei dem Implantat, welches in den Schenkelhals eines Femurs eingesetzt werden soll.

## Patentansprüche

1. Prophylaxe-Implantat gegen Frakturen osteoporotisch befallener Knochensegmente, insbesondere des Schenkelhalses, der Wirbelsäule und des Handgelenkes, aufweisend einen dünnwandigen hohlen Armierungskörper (1) mit einer Anzahl von Durchbrechungen (2) in seiner Außenwandung (3), **dadurch gekennzeichnet, daß** die Anzahl der Durchbrechungen (2) so groß ist, daß das Verhältnis der Gesamtflächen der Durchbrechungen (2) zu der Gesamtoberfläche wenigstens 1:2 beträgt.

2. Implantat nach Anspruch 1, bei dem der Armierungskörper (1) aus körperverträglichem Metall besteht und bei dem abschnittsweise eine offenmaschige, dreidimensionale Raumnetzstruktur (4) auf seiner Oberfläche vorgesehen ist.

3. Implantat nach Anspruch 1, bei dem der Armierungskörper (1) aus einem körperverträglichen Kunststoff besteht, wobei der Armierungskörper (1) mittels eines in ihm integrierten Mechanismus unter Aufbringung einer Zugkraft stauchbar ist.

4. Prophylaxe-Implantat nach einem der Ansprüche 1 bis 3 als Schenkelhalsimplantat, bei dem der Armierungskörper (1) aus einer im wesentlichen zylindrischen Hülse (5) besteht, welche distal mit einem balligen Kopfteil (6) verschlossen ist und welche proximal mit einem Verschlußstück (7) verschließbar ist (Fig. 1).

5. Implantat nach Anspruch 4 und Anspruch 2, bei dem das ballige Kopfteil (6) wenigstens bereichsweise mit der Raumnetzstruktur (4) versehen ist (Fig. 1).

6. Implantat nach Anspruch 5, bei dem das proximale Verschlußstück (7) wenigstens bereichsweise mit der Raumnetzstruktur (4) versehen ist (Fig. 1).

7. Implantat nach Anspruch 4 und Anspruch 3, bei dem im Inneren der Hülse (5) zwischen dem Kopfteil (6) und dem Verschlußstück (7) mit diesen verbunden eine Gewindespindel (8) verläuft, mittels der die Zugkraft auf deren Drehung erzeugbar ist (Fig. 3).

8. Implantat nach Anspruch 4 und Anspruch 3, bei dem in Inneren der Hülse (5) zwischen dem Kopfteil (6) und dem Verschlußstück (7) ein Zugseil (9) gespannt ist, das von extern weiter spannbar ist, um die zur Stauchung der Hülse (5) aufzuwendende Zugkraft erzeugen zu können (Fig. 4).

9. Implantat nach einer der Ansprüche 1 bis 8, bei dem die Außenwandung (3) mit einem vom Körper resorbierbaren Mittel (10) beschichtet ist (Fig. 1, 2).

10. Implantat nach Anspruch 9, bei dem das Mittel (10) Hydroxylapatit ist.

11. Implantat nach Anspruch 9, bei dem Mittel (10) aus einer alpha-Tricalciumphosphat-Keramik besteht.

12. Implantat nach einer der Ansprüche 1 bis 11, bei dem im Armierungskörper (1) auf einem darin zentriert angeordneten magnetischen Kern (30) eine elektrische Spule (31) angeordnet ist, die von extern anregbar ist.

13. Implantat nach Anspruch 12 und insbesondere nach Anspruch 7, bei dem der magnetische Kern (30) als Gewindespindel ausgebildet ist.

14. Implantat nach Anspruch 12 oder 13, bei dem das Kopfteil (6) des Armierungskörpers einen in das Innere des Armierungskörpers (1) ragenden Fortsatz (33) mit einer die zentrierte Lage des magnetischen Kerns (30) sichernden Aufnahme (32)aufweist.

## Claims

1. Prophylactic implant against fractures of bone segments affected by osteoporosis, in particular the femoral neck, the vertebral column and the wrist joint, having a thin-walled hollow reinforcing body (1) having a number of perforations (2) in its outer wall (3), **characterised in that** the number of perforations (2) is so large that the ratio of the total surfaces of the perforations (2) to the total surface area is at least 1:2.

2. Implant according to claim 1, in which the reinforcing body (1) consists of biocompatible metal and in which an open-meshed, three-dimensional spatial network structure (4) is provided in sections on its surface.

3. Implant according to claim 1, in which the reinforcing body (1) consists of a biocompatible plastic, wherein the reinforcing body (1) can be compressed by means of a mechanism integrated in it while applying a tensile force.

4. Prophylactic implant according to one of claims 1 to 3 as femoral neck implant, in which the reinforcing body (1) consists of an essentially cylindrical sleeve (5), which is closed distally by a spherical head part (6) and which can be closed proximally by a closure piece (7) (Figure 1).

5. Implant according to claim 4 and claim 2, in which the spherical head part (6) is provided with the spatial network structure (4) at least in some regions (Figure 1).

6. Implant according to claim 5, in which the proximal closure piece (7) is provided with the spatial network structure (4) at least in some regions (Figure 1).

7. Implant according to claim 4 and claim 3, in which a threaded spindle (8), by means of which the tensile force can be produced on rotation thereof, runs in the interior of the sleeve (5) between the head part (6) and the closure piece (7) connected to the latter (Figure 3).

8. Implant according to claim 4 and claim 3, in which a traction rope (9), which can be stretched further externally, in order to be able to produce the tensile force to be used to compress the sleeve (5), is stretched in the interior of the sleeve (5) between the head part (6) and the closure piece (7) (Figure 4).

9. Implant according to one of claims 1 to 8, in which the outer wall (3) is coated with an agent (10) which can be resorbed by the body (Figures 1, 2).

10. Implant according to claim 9, in which the agent (10) is hydroxylapatite.

11. Implant according to claim 9, in which agent (10) consists of an alpha-tricalcium phosphate ceramic.

12. Implant according to one of claims 1 to 11, in which an electric coil (31), which can be activated externally, is arranged in the reinforcing body (1) on a magnetic core (30) arranged centered therein.

13. Implant according to claim 12 and in particular according to claim 7, in which the magnetic core (30) is designed as a threaded spindle.

14. Implant according to claim 12 or 13, in which the head part (6) of the reinforcing body has an extension (33) projecting into the interior of the reinforcing body (1) and having a recess (32) securing the centered position of the magnetic core (30).

## Revendications

1. Implant à fonction prophylactique destiné à prévenir les fractures des segments osseux atteints d'ostéoporose, en particulier du col du fémur, de la colonne vertébrale et du poignet, comprenant un corps d'armature (1) muni d'une pluralité d'ajours (2) réalisés dans sa paroi extérieure (3), **caractérisé en ce que** le nombre des ajours (2) est si grand que le rapport de la superficie totale des ajours (2) sur la surface totale est au moins égal à 1:2.

2. Implant selon la revendication 1, dans lequel le corps d'armature (1) est réalisé dans un métal toléré par le corps humain et dans lequel il est prévu de réaliser dans certaines parties de sa surface une structure réticulaire à mailles ouvertes (4) développée dans un espace tridimensionnel.

3. Implant selon la revendication 1, dans lequel le corps d'armature (1) est réalisé dans une matière synthétique tolérée par le corps humain, le corps d'armature (1) pouvant être comprimé par l'application d'une force de traction au moyen d'un mécanisme intégré dans ledit corps d'armature.

4. Implant à fonction prophylactique selon l'une quelconque des revendications 1 à 3, conçu sous forme d'implant pour le col du fémur, dans lequel le corps d'armature (1) est formé par un fourreau (5) sensiblement cylindrique, qui est fermé sur le côté distal par une partie de tête convexe (6) et qui peut être fermé sur le côté proximal par une pièce d'obturation (7) (figure 1).

5. Implant selon la revendication 4 et la revendication 2, dans lequel la partie de tête convexe (6) est munie au moins dans certaines zones d'une structure réticulaire développée dans l'espacé (4) (figure 1).

6. Implant selon la revendication 5, dans lequel la pièce d'obturation (7) du côté proximal est munie au moins dans certaines zones d'une structure réticulaire développée dans l'espace (4) (figure 1).

7. Implant selon la revendication 4 et la revendication 3, dans lequel, à l'intérieur du fourreau (5), entre la partie de tête (6) et la pièce d'obturation (7) s'étend une broche filetée (8), assemblée avec cette dernière et par la rotation de laquelle il est possible de produire la force de traction (figure 3).

8. Implant selon la revendication 4 et la revendication 3, dans lequel un câble de traction (9) est tendu à l'intérieur du fourreau (5), entre la partie de tête (6) et la pièce d'obturation (7), lequel câble peut être tendu davantage à partir de l'extérieur, afin de pouvoir produire la force de traction à appliquer pour la compression du fourreau (5) (figure 4).

9. Implant selon l'une quelconque des revendications 1 à 8, dans lequel la paroi extérieure (3) est revêtue d'une substance (10) qui peut être résorbée par le corps humain (figures 1, 2).

10. Implant selon la revendication 9, dans lequel la substance (10) est une apatite hydroxyle.

11. Implant selon la revendication 9, dans lequel la substance (10) est formée par une céramique à phosphate de tricalcium alpha.

12. Implant selon l'une quelconque des revendications 1 à 11, dans lequel une bobine électrique (31) est disposée sur un noyau magnétique (30), disposé au centre du corps d'armature (1), laquelle bobine peut être sollicitée par une excitation externe.

13. Implant selon la revendication 12 et en particulier la revendication 7, dans lequel le noyau magnétique (30) est réalisé sous forme de broche filetée.

14. Implant selon la revendication 12 ou 13, dans lequel la partie de tête (6) du corps d'armature est munie d'un prolongement (33) qui s'engage à l'intérieur du corps d'armature (1) et qui comporte un évidement (32) destiné à assurer la position centrale du noyau magnétique (30).
